# EUROPEAN PATENT SPECIFICATION

(11) **EP 1 285 647 B1**
(45) Date of publication and mention of the grant of the patent: **04.08.2010**
(21) Application number: 02255724.3
(22) Date of filing: 16.08.2002
(51) Int. Cl.: A61K 8/43, A61Q 5/10, A61Q 5/12

(54) **Hair cosmetic comprising guanidine derivative and N-alpha-acylarginine**
Kosmetisches Haarpflegemittel enthaltend ein Guanidinderivat und ein N-alpha-Acylarginin
Produit capillaire cosmétique contenant un dérivé de guanidine et une N-alpha-acylarginine

(30) Priority: 21.08.2001 JP 2001250558
(43) Date of publication of application: 26.02.2003
(73) Proprietor: Ajinomoto Co., Inc., Kanagawa 210-8681 (JP)
(72) Inventor: Yumioka, Ryosuke, c/o Ajinomoto Co., Inc., Kawasaki-ku, Kawasaki-shi 210-8681 (JP); Yokota, Hirofumi, c/o Ajinomoto Co., Inc., Kawasaki-ku, Kawasaki-shi 210-8681 (JP)
(74) Representative: Nicholls, Kathryn Margaret

(56) References cited:
- US-A- 4 975 275
- PATENT ABSTRACTS OF JAPAN vol. 1999, no. 13, 30 November 1999 (1999-11-30) & JP 11 228348 A (AJINOMOTO CO INC), 24 August 1999 (1999-08-24)
- PATENT ABSTRACTS OF JAPAN vol. 1999, no. 13, 30 November 1999 (1999-11-30) & JP 11 228527 A (AJINOMOTO CO INC), 24 August 1999 (1999-08-24)
- PATENT ABSTRACTS OF JAPAN vol. 2000, no. 22, 9 March 2001 (2001-03-09) & JP 2001 139433 A (LION CORP), 22 May 2001 (2001-05-22)

## Description

The present invention relates to a hair cosmetic embodiments of which may provide conditioning properties such as smoothness in rinsing, excellent finger passing when wet, and settlement of hair when dry.

Hair cosmetics are composed of various raw materials such as surfactants, oily raw materials, wetting agents, polymer compounds, preservatives and bacteriocides, antioxidants, ultraviolet absorbers, coloring agents, aromatics and the like.

Conventionally, as the cationic surfactant in a hair cosmetic, quaternary ammonium salts are generally used. However, quaternary ammonium salts have demerits that adsorption to hairs is weak due to steric hindrance since a charged nitrogen atom which is an adsorption center to hairs is covered with a long chain or short chain alkyl group or alkenyl group, and the salts are flown in rinsing since water-solubility is high. Therefore, hair cosmetics using quaternary ammonium salts are not necessarily satisfactory from the standpoints of conditioning properties such as smoothness in rinsing, excellent finger passing when wet, and settlement of hair when dry.

Recently, amide group-containing guanidine derivatives and salts thereof are utilized as a cationic surfactant in a hair cosmetic, instead of general purpose quaternary ammonium salts. It is known that a hair cosmetic using an amide group-containing guanidine derivative or salt thereof shows excellent adsorption property to hairs as compared with quaternary ammonium salts, and gives hair excellent flexibility and wetting property, and excellent finished feeling (Japanese Patent Application Laid-Open No.2-243614, 4-49221 and 4-49222).

Thus, use of an amide group-containing guanidine derivative in a hair cosmetic is already widely known. However, when an amide group-containing guanidine derivative is used as a hair cosmetic, there is a problem that, though smoothness in rinsing is improved as compared with a quaternary ammonium salt, the amide group-containing guanidine derivative still shows high water-solubility and flows in rinsing since the derivative is usually used in the-form of salt. Therefore, the amide group-containing guanidine derivative has not been sufficiently satisfactory in conditioning properties such as excellent finger passing when wet and settlement of hair when dry.

Desirably, embodiments of the hair cosmetic of the present invention may provide excellent conditioning properties such as smoothness in rinsing, excellent finger passing when wet, and settlement of hair when dry.

The present inventors have intensively studied in view of these desiderata, and resultantly found that a hair cosmetic embodiments of which may have excellent conditioning properties such as smoothness in rinsing, excellent finger passing when wet, and settlement of hair when dry may be obtained, if one more components selected from amide group-containing guanidine derivatives and salts thereof and one more components selected from Nα-acylarginines and salts thereof are used in combination in a hair cosmetic, leading to completion of the present invention.

Namely, the hair cosmetic of the present invention comprises, at least, one or more components A selected from amide group-containing guanidine derivatives of the following general formula (I), 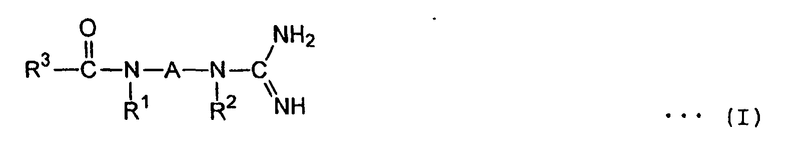 (in the general formula (I), R¹ and R² represent a hydrogen atom, or a linear or branched alkyl or alkenyl group having 1 to 4 carbon atoms, and may be the same or different. R³ represents a linear or branched alkyl or alkenyl group having 1 to 22 carbon atoms . A represents a linear or branched alkylene or alkenylene group having 1 to 10 carbon atoms.) and salts thereof, and one or more components B selected from Nα-acylarginines of the following general formula (II), 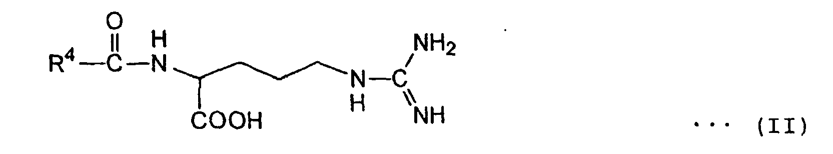 (in the general formula (II), R⁴ represents a linear or branched alkyl or alkenyl group having 1 to 22 carbon atoms.)
and salts thereof.

It is known that the Nα-acylarginines and salts thereof (component B) compounded as essential components in the hair cosmetic of the present invention are used in combination of quaternary ammonium salts of cationic surfactants as components of a cosmetic composition (Japanese Patent Application Laid-Open No. 11-228348), however, there is no example reported investigating a combination with an amide group-containing guanidine derivative and salt thereof (component A).

In embodiments of the present invention, conditioning properties such as smoothness in rinsing, excellent finger passing when wet, and settlement of hair when dry may be improved overall, by using one or more components A selected from amide group-containing guanidine derivatives and salts thereof and one or more components B selected from Nα-acylarginines and salts thereof, in combination.

These and other objects, features and advantages of the present invention are specifically set forth in or will become apparent from the following detailed descriptions of embodiments of the invention

The hair cosmetic of the present invention comprises one or more components A selected from amide group-containing guanidine derivatives and salts thereof and one or more components B selected from Nα-acylarginines and salts thereof, as essential components.

The hair cosmetic of the present invention will be described in detail in this order, below.
[I] Amide group-containing guanidine derivative (component A),
[II] Nα-acylarginine (component B), and
[III] Hair cosmetic,

### [I] Amide group-containing guanidine derivative

The amide group-containing guanidine derivative used in the present invention is represented by the following general formula (I).

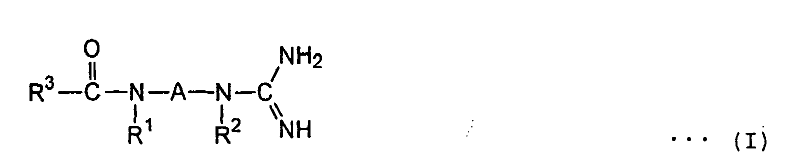

In the general formula (I), R¹ and R² represent a hydrogen atom, or a linear or branched alkyl or alkenyl group having 1 to 4 carbon atoms. R¹ and R² may be the same or different. R¹ and R² represent most preferably a hydrogen atom.

As the acyl group (R³CO) in the general formula (I), linear or branched and saturated or unsaturated acyl groups having 1 to 22 carbon atoms are listed. As the specific examples of the acyl group (R³CO), acyl groups derived from acetic acid, propionic acid, capric acid, lauric acid, myristic acid, palmitic acid, stearic acid, behenic acid, linoleic acid, linolenic acid, oleic acid, isostearic acid, 2-ethylhexanoic acid, coconut oil fatty acid, beef tallow fatty acid, hardened beef tallow fatty acid and the like are listed. As the preferably acyl group, a caproyl group, lauroyl group, myristyl group, palmitoyl group, stearoyl group, behenoyl group, cocoyl group, hardened beef tallow fatty acyl group and the like are listed, and further preferably, a lauroyl group, myristyl group, palmitoyl group and stearoyl group are listed.

In the general formula (I), A represents a branched or linear alkylene group or alkenylene group having 1 to 10 carbon atoms, preferably 2 to 6 carbon atoms . As the specific examples of A, an ethylene group, propylene group, butylene group, pentylene group and hexylene group are listed.

The amide group-containing guanidine derivative is compounded, usually in the form of salt, in a hair cosmetic. Specifically, the amide group-containing guanidine derivative can be used in the form of inorganic acid salts such as a hydrochloride, sulfate, phosphate and the like, or organic acid salts such as a glycolate, acetate, citrate, acidic amino acid salt, pyrrolidonecarboxylate, and the like.

The amide group-containing guanidine derivative or salt thereof used in the present invention can be obtained as described below. A monoaminde amine derived from a diamine is subjected to heating pressure reduction treatment, heating nitrogen-bubbling treatment, or stored under an atmosphere of no carbon dioxide, then, guanidylated with cyanamide, S-methylisothiourea and the like, further, mixed impurities are removed by means such as crystallization and the like, as disclosed, for example, in Japanese Patent Application Laid-Open No. 6-312972.

The amide group-containing guanidine derivative or salt thereof used in the present invention can also be produced by reacting an amino group-containing guanidine derivative of the following general formula (III) with a fatty acid halide or fatty acid ester. 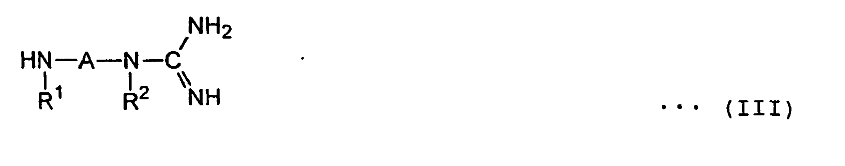 (in the general formula (III), R¹ and R² represent a hydrogen atom, or a linear or branched alkyl or alkenyl group having 1 to 4 carbon atoms, and may be the same or different. A represents a linear or branched alkylene or alkenylene group having 1 to 10 carbon atoms.).

In producing an amide group-containing guanidine derivative, it is also preferable that arginine is previously mixed into an amino group-containing guanidine derivative represented by the general formula (III), and this mixture is reacted with a fatty acid halide or fatty acid ester. Here, arginine is a raw material of the Nα-acylarginine (component B) which is another essential component of the hair cosmetic of the present invention, and in this case, since an amide group-containing guanidine derivative (component A) and Nα-acylarginine (component B) can be obtained simultaneously as reaction products, the resulted reaction products can be utilized as they are in the hair cosmetic of the present invention.

### [II] Nα-acylarginine

The Nα-acylarginine (component B) will be described in detail below.

The Nα-acylarginine in the present invention is represented by the following general formula (II). 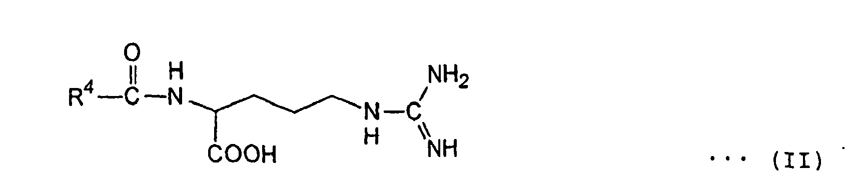 (in the general formula (II), R⁴ represents a linear or branched alkyl or alkenyl group having 1 to 22 carbon atoms.).

The Nα-acylarginine used in the present invention is a compound derived from arginine. The Nα-acylarginine has a guanidine group like the amide group-containing guanidine derivative or salt thereof (component A), and used as an amphoteric surfactant in various applications such as cosmetic compositions, lubricants, antistatic agents, surface treating agents and the like.

Use of the Nα-acylarginine in combination with a quaternary ammonium salt of a cationic surfactant, as a component of a cosmetic composition, has been already investigated, as disclosed in Japanese Patent Application Laid-Open No. 11-228348, however, there is no example known investigating a combination with an amide group-containing guanidine derivative.

The Nα-acylarginine in the present invention has a linear or branched and saturated or unsaturated fatty acyl group having 1 to 22 carbon atoms, and specific examples thereof include Nα-acetylarginine, Nα-propionylarginine, Nα-octanoylarginine, Nα-decanoylarginine, Nα-lauroylarginine, Nα-myristoylarginine, Nα-palmitoylarginine, Nα-stearoylarginine, Nα-behenoylarginine, Nα-coconut oil fatty acid acylarginine, Nα-palm kernel oil fatty acid acylarginine, Nα-beef tallow fatty acid acylarginine and the like. Of them, Nα-lauroylarginine, Nα-myristoylarginine, Nα-palmitoylarginine and Nα-stearoylarginine are preferable from the standpoint of touch feeling, and Nα-lauroylarginine, Nα-myristoylarginine and Nα-palmitoylarginine are more preferable.

The Nα-acylarginine which can be used in the present invention can be obtained, as described in Japanese Patent Application Laid-Open No. 48-23729, by reacting arginine which is one of amino acids with a fatty acid halide under alkaline condition together with a hydrophilic organic solvent, or, as described in Japanese Patent Application Laid-Open No. 49-1513, by thermally dehydrating a salt of arginine and a fatty acid at temperatures of 100 to 250°C. Further, a crystal of a Nα-acylarginine can be obtained, as shown in Japanese Patent Application Laid-Open No. 11-228527, by reacting arginine and a fatty acid halide in the presence of an alkali (pH 10 to 13) using a mixed solvent of lower alcohol and/or polyhydric alcohol and water, then, converting the reaction solution into an acidic or basic aqueous solution to dissolve the product, then, controlling pH to 5 to 7. As the arginine used as a raw material of a Nα-acylarginine, any of D form, L form and DL form can be used.

The Nα-acylarginine may be compounded in the form of salt into a hair cosmetic. Specifically, the Nα-acylarginine can be used in the form of inorganic acid salts such as a hydrochloride, sulfate, phosphate and the like, or organic acid salts such as a glycolate, acetate, citrate, acidic amino acid salt, pyrrolidonecarboxylate, and the like.

### [III] Hair cosmetic

Into the hair cosmetic of the present invention, various additives usually used can be added as optional components in an amount not disturbing the effect of the present invention, in addition to the above-described component A and component B. For example, raw materials described in the cosmetic raw material standard, cosmetic type compounding component standard, medicine raw material standard, Japanese pharmacopeia and Japanese Standard of Food Additives, such as surfactants, wetting agents, silicone compounds, polymer substances (polymer compounds), alcohols, ultraviolet absorbers, coloring matters, pigments, vitamins, antioxidants, sequestering agents, preservatives, bactericides, pH controlling agents, pearlescent agents, nucleic acids, enzymes, natural extracts and the like, are listed.

Examples of the surfactant include the following compounds.

### 1. Anionic surfactant

alkylsulfates, alkylphosphates, polyoxyethylene alkyl ether sulfates, polyoxyethylene alkylphenyl ether sulfates, polyoxyethylene alkylcarboxylates, alkylphenyl ether sulfonates, salts of alkylsulfosuccinic acids and derivatives thereof, salts of N-acylsarcosine and derivatives thereof, salts of N-acyl-N-methyl-β-alanine, polyoxyethylene coconut oil fatty acid monoethanolamide sulfate, polyoxyethylene alkyl ether phosphates, higher fatty acid salts, salts of N-acylglycine, salts of N-acylalanine and the like.

### 2. Amphoteric surfactants

carbobetaine type amphoteric surfactants, sulfobetaine type amphoteric surfactants, hydroxysulfobetaine type amphoteric surfactants, amidesulfobetaine type amphoteric surfactants, phosphobetaine type amphoteric surfactants, imidazoline type amphoteric surfactants, lecithin derivatives and the like.

### 3. Nonionic surfactants

propylene glycol fatty acid esters, glycerine fatty acid esters, polyoxyethylene glycerine fatty acid esters, polyglycerine fatty acid esters, sorbitan fatty acid esters, polyoxyethylene sorbitan fatty acid esters, polyoxyethylene sorbitol fatty acid esters, polyoxyethylene alkylphenyl formaldehyde condensates, polyoxyethylene castor oil, polyoxyethylene hardened castor oil, polyoxyethylene sterol and derivatives thereof, polyethylene glycol fatty acid esters, polyoxyethylene alkyl ethers, polyoxyethylene polyoxypropylene alkyl ethers, polyoxyethylene alkylphenyl ethers, polyoxyethylene lanolin and derivatives thereof, polyoxyethylene beeswax derivatives, polyoxyethylene alkylamines, polyoxyethylene fatty acid amides, alkanolamides, sugar esters, polyoxyethylene hardened castor oil pyroglutamates, polyoxyethylene glyceryl pyroglutamates, and the like

### 4. Cationic surfactants

quaternary ammonium salts, amideamines, salts of N-acylarginine esters, salts of N-[3-alkyloxy-2-hydroxypropyl]-L-arginine, and the like.

As the salts of anionic surfactants, sodium salts, magnesium salts, potassium salts, ammonium salts, diethanolamine salts, triethanolamine salts, arginine salts, lysine salts and the like are listed, and these various surfactants can be added.

Examples of the wetting agent include the following compounds.

### 1. Polyhydric alcohols

glycerine, propylene glycol, 1,3-butylene glycol, isoprene glycol, polyethylene glycol, diglycerine, triglycerine, tetraglycerine, pentaglycerine, hexaglycerine, decaglycerine, sorbitol, ethylene glycol, erythritol and the like

### 2. Natural moistening factor (NMF)

amino acids such as proline, glycine, alanine, serine, threonine, arginine, glutamic acid, aspartic acid, leucine, isoleucine, valine and the like, pyrrolidonecarboxylic acid or pyrrolidonecarboxylates, lactic acid or lactates, urea, glucosamine, creatine, citrates, and the like

### 3. Saccharides, polysaccharides and polysaccharide derivatives

scrose, lactose, trehalose, natural oligosaccharides, hyaluronic acid and sodium salts thereof, chondroitin sulfate, and the like

### 4. Polyamino acid

polyamino acids including polyglutamic acid and polyaspartic acid, and salt thereof, additionally, amino acid derivatives, soybean protein hydrolyzate, deoxyribonucleic acid, glycinebetaine, chitin, chitosan, deacetylated chitin, and the like

Examples of the silicone compound include the following compounds.

### 1. Ether-modified silicone

methyl polysiloxane, highly polymerized methyl polysiloxane, polyoxyethylene-methyl polysiloxane copolymer, polyoxypropylene-methyl polysiloxane copolymer and poly(oxyethylene, oxypropylene)-methylpolysiloxane copolymer, and the like

### 2. Amino-modified silicone

stearoxy methyl polysiloxane, stearoxy trimethylsilane, methyl hydrogen polysiloxane, octamethyl polysiloxane, decamethyl polysiloxane, cyclic silicones such as decamethyl cyclopentasiloxane, octamethyl cyclotetrasiloxane, tetrahydrotetramethyl cyclotetrasiloxane, methyl cyclopolysiloxane, dodecamethyl cyclohexasiloxane and the like, methyl phenyl polysiloxane, trimethyl siloxy silic acid, aminoethyl aminopropyl siloxane-dimethyl siloxane copolymer, and the like

### 3. Others

silanol-modified polysiloxane, alkoxy-modified polysiloxane, fatty acid-modified polysiloxane, fluorine-modified polysiloxane, epoxy-modified polysiloxane, alkoxy-modified polysiloxane perfluoro polyether, polyvinyl acetate dimethyl polysiloxane and the like. In addition, silicone emulsions prepared by emulsifying one or two or more of them, and the like can also be used.

Examples of the polymer substance (polymer compound) include the following compounds.

### 1. Vegetable-based polysaccharides polymers

guar gum, locust bean gum, queens seed, carageenan, galactan, gum Arabic, tragacanth rum, pectin, mannan, starch, pullulan and the like

### 2. Polysaccharides-based polymers derived frommicroorganisms

xanthan gum, dextran, succinoglucan, curdlan, hyaluronic acid and the like

### 3. Animal-based proteins

gelatin, casein, albumin, collagen and the like 4. Cellulose derivatives
methyl cellulose, ethyl cellulose, hydroxyethyl cellulose, hydroxypropyl cellulose, carboxymethyl cellulose and salts thereof, methyl hydroxypropyl cellulose and the like 5. Starch derivatives
soluble starch, carboxyl starch, methyl starch and the like

### 6. Alginic acid derivatives

propylene glycol alginate ester, alginate and the like 7. Vinyl-based derivatives

polyvinyl alcohol, polyvinyl butyral, vinyl acetate-vinylpyrrolidone copolymer, vinyl acetate-crotonic acid copolymer, vinyl methyl ether-ethyl maleate copolymer, vinyl methyl ether-butyl maleate copolymer, crotonic acid-vinyl acetate-vinyl neodecanoic acid copolymer, methoxyethylene maleic anhydride copolymer, isobutylene-sodium maleate copolymer, N-methylpyrrolidone, vinylpyrrolidone-N, N-dimethylaminoethylmethacrylic acid copolymer diethyl sulfate salt, vinylimidazolium methochloride-vinylpyrrolidone copolymer, polyvinyl pyrrolidone, vinyl pyrrolidone-styrene copolymer, vinyl pyrrolidone-hexadecene copolymer, styrene-vinyl pyrrolidone copolymer, eicosene-vinyl pyrrolidone copolymer, carboxyl vinyl polymer and the like

### 8. (meth)acrylic acid derivatives

alkyl acrylate copolymers, polyacrylic acid and salts thereof (sodium, potassium, ammonium, triethanolamine, arginine, lysine, and the like), polyalkyl acrylates, alkyl acrylate copolymers, acrylic amide-styrene copolymers, alkyl acrylate-styrene copolymers, octyl acrylate amide-acrylate copolymers and salts thereof, octyl acrylate amide-hydroxypropyl acrylate-butyl methacrylate aminoethyl copolymers, acrylic resin alkanolamine, hydroxyethyl acrylate-methoxyethyl acrylate copolymer, alkyl acrylate-alkyl methacrylate-diacetone-acetoneacrylamide-methacrylic acid copolymer, dimethyl chloride diallyl ammonium-acrylamide copolymer and the like, methacryloyl ethyl dimethyl betaine-methacryloyl chloride ethyl trimethyl ammonium-methoxy methacrylate polyethylene glycol copolymer, methacryloyl ethyl dimethyl betaine-methacryloyl chloride ethyl trimethyl ammonium-2-hydroxyethyl methacrylate copolymer

### 9. Cationated cellulose

O-[2-hydroxy-3-(trimethylammonio)propyl]hydroxyethy 1 cellulose chloride, O-[2-hydroxy-3-(lauryldimethylammonio)propyl]hydroxyethyl cellulose chloride and the like

### 10. Cationated guar gum

O-[2-hydroxy-3-(trimethylammonio)propyl]guar gum chloride, and the like

### 11. Others

Carboxymethyldextran and salts thereof, epoxy resin isostearic acid esters, polyamide epichlorohydrin resins, bisphenol epoxy resin fatty acid esters, polyethylene glycol-epichlorohydrin-coconut oil alkylamine-dipropylenetriamine condensate, perfluoro polyether and the like

Examples of the alcohol include higher alcohols such as cetyl alcohol, stearyl alcohol, behenyl alcohol, isostearyl alcohol, octyl dodecanol, oleyl alcohol, myristyl alcohol and the like, lower alcohols such as ethanol, isopropyl alcohol and the like.

Examples of the ultraviolet absorber include benzophenone derivatives, p-aminobenzoic acid derivatives, methoxy cinnamic acid derivatives,salicylic acid derivatives, urocanic acid and derivatives thereof, 4-tert-butyl-4'-methoxydibenzoylmethane, 2-(2'-hydroxy-5'-methylphenyl)benzotriazole, methyl anthranilate and the like.

Examples of the coloring matter include Red 201, Red 202, Red 204, Red 205, Red 220, Red 226, Red 228, Red 405, Orange 203, Orange 204, Yellow 205, Yellow 401, Blue 404, Red 3, Red 104, Red 106, Red 227, Red 230, Red 401, Red 505, Orange 205, Yellow 4, Yellow 5, Yellow 202, Yellow 203, Green 3, Blue 1 and the like.

Examples of the pigment include natural coloring matters such as β-carotene, calsamine, cochineal and the like, and inorganic pigments such as mica, talc, kaolin, calcium carbonate, magnesium carbonate, anhydrous silic acid, aluminum oxide, barium sulfate, red iron oxide, yellow iron oxide, black iron oxide, chromium oxide, ultramarine, iron blue carbon black, titanium oxide, zinc oxide, mica titanium, squama foil, bismuth oxychloride, boron nitride, photochromic pigment, synthetic fluorine gold mica, iron-containing synthetic fluorine gold mica, fine particle complex powder and the like.

Examples of the vitamin include vitamins A, B1, B2 and B6 and derivatives thereof used as a nutrient, vitamin C and derivatives thereof used as a whitening agent, vitamin E and derivatives thereof used as a vasodilator, and the like.

Examples of the antioxidant include tocophenols, dibutyl hydroxy toluene (BHT), butyl hydroxy anisole (BHA) and the like.

Examples of the sequestering agent include sodium ethylenediamine tetraacetate (EDTA), phosphoric acid, citric acid, succinic acid, gluconic acid, polyphosphoric acid, sodium metaphosphate and the like.

Examples of the preservative and bacteriocide include benzoic acid, sodium benzoate, isopropyl methyl phenol, zinc undecylenate, undecylene monoethanolamide, zinc chloride, benzalkonium chloride, alkyl trimethyl ammonium chloride, cetyl pyridinium chloride, benzalkonium chloride, alkyldiaminoethylglycine hydrochloride, chlorhexidine hydrochloride, O-phenyl phenol, photosensitive element 101, photosensitive element 201, chlorhexidine gluconate, cresol, chloramine T, chlorxylenol, chlorcresol, chlorphenesine, chlorobutanol, salicylic acid, sodium salicylate, alkyl isoquinoliniumbromide, domiphenbromide, sorbic acid and salts, thymol, thiram, dehydroacetic acid and sodium salt, trichloro carbanilide, p-oxy benzoate, chlorophenol, halocarbene, phenol, hexachlorophen, resorcin, triclosan, benzethonium chloride and the like.

Additionally, swertia herb extract, cepharanthin, γ -orizanol, capsicum tincture, ginger tincture and benzyl nicotinate are listed as a hair restoration drug, estradiol and ethynyl estradiol are listed as a female hormone agent, and pantothenic acid and derivatives thereof, placenta extract, allantoin, photosensitive element 301 and the like are listed as a hair root activating agent.

As the drug derived from natural substances, hamamelis, Lamium album, Betula alba, rheum, licorice, coptis, tibouchina, yarrow, aloe, chamomile, refined oils such as eucalyptus oil, carrot extract, aloe, tibouchina, lilac, Luffa, marronnier, fleawort, Safflower and the like are listed.

Production of the hair cosmetic of the present invention by compounding various components described above can be conducted according to a conventionally known method without specific restriction excepting the component A and the component B are used as essential components.

The compounding amount of the component A in the present invention is appropriately determined depending on the intended product, and is not particularly restricted, and preferably from 0.001 to 10% by weight, particularly preferably from 0.1 to 10% by weight. When less than 0.001% by weight, the effect of the present invention is not manifested sufficiently in some cases.

The compounding amount of the component B in the present invention is appropriately determined depending on the intended product, and is not particularly restricted, and preferably from 0.001 to 3% by weight, more preferably from 0.001 to 1% by weight.

The compounding ratio of the component A to the component B is appropriately determined depending on the intended product, and is not particularly restricted, and preferably from 200/1 to 1/10 (= component A/component B, by weight), particularly preferably from 200/1 to 1/1 (by weight).

The hair cosmetic of the present invention is not particularly restricted in its dosage form, and any dosage forms are permissible such as an emulsion system, solution system, solubilization system, powder dispersion system, water-oil two layer system, water-oil-powder three layer system and the like.

The hair cosmetic of the present invention is not particularly restricted also in its application, and can be applied to shampoo, rinse, hair restoration agent, treatment, hair conditioner, tick, hair liquid, set lotion, permanent wave liquid, hair cream, hair lotion, hair mousse, permanent liquid, hair dye, hair color, hair manicure, and the like.

### Example 1, Comparative Examples 1 to 3

The following comparative examples and examples further explain embodiments of the present invention.

### (1) Synthesis of amide group-containing guanidine derivative (component A)

180g of water and 110g of 2-propanol were added to 52.7g (0.23mol) of 4-aminobutylguanidine sulfate. pH of the system was adjusted to be 11.0 by 27% sodium-hydroxide aqueous solution and the temperature was adjusted to be 10 °C. In the system, 50.3g (0.23mol) of lauroyl chloride was added dropwise over 35minutes. At this time, the temperature of the system was kept at 8 to 12 °C, and pH was kept at 10.9 to 11.0 by the 27% sodium hydroxide aqueous solution. After completion of the dropwise addition, the reaction was further ripened over 30minutes.

After completion of reaction, the temperature of the system was adjusted to be 50 °C. The resulting oily phase was separated, adjusted to be pH 14.0 by 27% sodium hydroxide aqueous solution and cooled to room temperature. After cooling, the precipitated solid was separated by filtration and after it was further washed with water, it was dried.
Light yellow solid 65.8g (yield 92%)
¹H-NMR(CD3OD) : σ = 0.90(3H, t), 1.28-1.38(18H, m), 1.57(6H, m), 2.17(2H, t), 3.19(4H, m)
ESI-Mass: 313(MH+)

A peak was confirmed by High Performance Liquid Chromatography (column: YMC-Pack ODS-AM AM-312, S-5um, 120A, 150*6.0mm, column temperature: 40 °C, eluent: 30mM Phosphate buffer (pH3.0) / methanol = 25/75 flow rate : 1.0ml/min, detection :UV210nm).

0.1% of sulfate ion in lauroylamidobutylguanidine was confirmed by Ion Chromatography (column: Ion pac AS-11 HC 2mm (Dionex), guard column: Ion pac AG-11 HC 2mm (Dionex), column temperature: room temperature, eluent: NaOH(aq) 1.5mM (0min) → 30mM(20min), flow rate:0.38ml/min, recycle solution: 5mM H₂SO₄ about 3mL/min, detection: conductivity) .

The amide group-containing guanidine derivatives of different acyl chain lengths used in the examples were also produced by the same method.

### (2) Synthesis of Nα-acylarginine (component B)

20 g (0.11 mol) of L-arginine was added to a mixture of 52 ml of water and 156 ml of isopropyl alcohol, and the temperature of the system was controlled to 10°C. 27.6 g (0.13 mol) of lauroyl chloride was dropped over 45 minutes while maintaining the temperature of the system at 10°C. In this operation, the temperature of the system was kept at 10 to 13°C and pH of the system was kept at 9.9 to 10.5 with a 27% sodium hydroxyl aqueous solution. After completion of dropping, the reaction was aged further over 30 minutes . After completion of the reaction, concentrated hydrochloric acid was added to control pH of the system to 3.8, and the temperature was controlled to 50°C for attaining dissolution. Then, pH of the system was controlled to 5.0 with a 27% sodium hydroxide aqueous solution, the system was cooled while stirring, and the precipitated solid was filtrated, and dried. From the identification results of ¹H-NMR, FAB-Mass and high performance liquid chromatography of the reaction product, production of lauroylarginine could be confirmed.
White solid, 31 g (yield: 78%)
¹H-NMR (CD₃OD), σ = 0.89 (3H, t), 1.24 to 1.38 (18H, m), 1.62 (2H, m), 1.78 (2H, m), 2.23 (2H, t), 3.11 (2H, m), 4.27 (1H, t)
FAB-Mass: 357 (MH+)

The peak was confirmed by high performance liquid chromatography (column: YMC-Pack ODS-AM AM-312, S-5um, 120A, 150*6.0 mm, column temperature: 40°C, eluant: 30 mM phosphate buffer (pH 3.0)/methanol = 25/75, flow rate: 1 ml/min, detection: UV 210 nm).

The Nα-acylarginines of different acyl chain lengths used in the examples were also produced by the same method.

### (3) Preparation of hair rinse and function evaluation

4 g of hair bundle (20 cm) was washed with about 1 ml of a 15% aqueous solution of sodium polyoxyethylene (3) lauryl ether sulfate (LES: manufactured by Kao Corporation., Emal 20 C), and rinsed for 2 minutes. 0.5 g (wt% of net composition shown in the following Table 1, total 100%) of hair rinse prepared by an ordinary method was taken, and made to contact with the hair under wet condition for 1 minute, then, rinsed for 1 minute, and smoothness of hair in rinse, excellent finger passing when wet, and settlement of hair when dry on the next day, were evaluated. These results are also described in the same table together.

Functional evaluation was carried out by four special panelists. Comparative Example 1 is the standard article, and when the average value calculated according to the following standards is 5.0 to 3.5, evaluation is very good ( 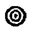 ), when 3.5 to 2.5, evaluation is normal (○), and when 2.5 to 1.0, evaluation is poor (Δ).

### <Evaluation standard>

(a) Smoothness in rinse
   5: smoother than standard article
   4: somewhat smooth more than standard article
   3: equivalent to standard article
   2: somewhat less smooth than standard article
   1: less smooth than standard article
(b) Excellent finger passing when wet
   5: more excellent finger passing than standard article
   4 : somewhat excellent finger passing more than standard article
   3: equivalent to standard article
   2 : somewhat less excellent finger passing than standard article
   1: less excellent finger passing than standard article
(c) Settlement of hair when dry
   5: more settled than standard article
   4: somewhat settled more than standard article
   3: equivalent to standard article
   2: somewhat less settled than standard article
   1: less settled than standard article

**Table 1**

| Hair rinse | | | | |
|---|---|---|---|---|
| | Comparative Example | | | Example |
| Component | 1 | 2 | 3 | 1 |
| (component A) | | | | |
| 4-guanidinobutyldodecaneamide acetate | | | 1.0 | 1.0 |
| (component B) | | | | |
| Nα-lauroylarginine | | 1.0 | | 1.0 |
| (other components) | | | | |
| Monobehenyl ammonium chloride (net 80%) * | 1.25 | 1.25 | | |
| Cetanol/stearyl alcohol (7/3) ** | 3.0 | 3.0 | 3.0 | 3.0 |
| Purification water | 95.75 | 95.75 | 96.00 | 96.00 |
| | | | | |
| (a) Smoothness in rinse | - | ⊚ | ⊚ | ⊚ |
| (b) Excellent finger passing when wet | - | Δ | ○ | ⊚ |
| (c) Settlement of hair when dry | - | Δ | ○ | ⊚ |

| | | | | |
|---|---|---|---|---|
| * Arcade 22-80 (manufactured by Lion Corporation.) ** Calchol 6870 (manufactured by Kao Corporation.) | | | | |

The hair rinse of Comparative Example 2 prepared by compounding the general purpose quaternary ammonium salt (monobehenyl ammonium chloride) and Nα-acylarginine (component B) in combination shows improvement in smoothness in rinse as compared with the standard article (Comparative Example 1), however, since the amide group-containing guanidine derivative (component A) is not contained, excellent finger passing when wet and settlement of hair when dry are rather poor. Also the hair rinse of Comparative Example 3 prepared by compounding the amide group-containing guanidine derivative (component A) shows improvement in smoothness in rinse as compared with Comparative Example 1, however, evaluation items such as excellent finger passing when wet and settlement of hair when dry are still not satisfactory.

In contrast, the hair rinse of Example 1 containing 4-guanidinobutyldodecaneamide acetate (component A) which is one of amide group-containing guanidine derivatives and Nα-lauroylarginine (component B) which is one of Nα-acylarginines is excellent overall in all evaluation items.

As described above, comparison of Example 1 with Comparative Examples 1 to 3 teaches that conditioning properties such as smoothness in rinse, excellent finger passing when wet and settlement of hair when dry can be improved by compounding the amide group-containing guanidine derivative (component A) and Nα-acylarginine (component B) in combination.

### Examples 2 to 5

Hair rinses having compositions (wt% of net composition, total 100%) shown in the following Table 2 were prepared. When the resulted hair rinses were used, excellent use feelings were obtained in conditioning properties such as smoothness in rinse, excellent finger passing when wet and settlement of hair when dry.

**Table 2**

| Hair rinse | | | | |
|---|---|---|---|---|
| Component | Example | | | |
| | 2 | 3 | 4 | 5 |
| (component A) | | | | |
| 4-guanidinobutylcocoylamide acetate | | | | 3.0 |
| 4-guanidinobutyldecaneamide hydrochloride | 3.0 | | | |
| 4-guanidinobutylmyristylami de glutamate | | | 3.0 | |
| 4-guanidinobutylpalmitylami de pyrrolidone carboxylate | | 3.0 | | |
| (component B) | | | | |
| Nα-2-ethylhexanoylarginine | | | 0.05 | |
| Nα-cocoylarginine | 0.1 | | | |
| Nα-myristoylarginine | | | 0.05 | |
| Nα-palmitoylarginine | | 0.1 | | 0.1 |
| (other components) | | | | |
| Sorbitol | 3.0 | 3.0 | 3.0 | 3.0 |
| Serine | 1.0 | 1.0 | 1.0 | 1.0 |
| Glyceryl octanoate | 3.0 | 3.0 | 3.0 | 3.0 |
| Octyldodecyl myristate | 3.0 | 3.0 | 3.0 | 3.0 |
| Setostearyl alcohol | 3.0 | 3.0 | 3.0 | 3.0 |
| Stearyldimethyl ammonium chloride | 2.0 | 2.0 | 2.0 | 2.0 |
| Polyoxyethylene stearyl ether | 0.5 | 0.5 | 0.5 | 0.5 |
| Preservative | 0.1 | 0.1 | 0.1 | 0.1 |
| Purification water | Residue | Residue | Residue | Residue |

### Examples 6 to 10

Hair treatments having compositions (wt% of net composition, total 100%) shown in the following Table 3 were prepared. When the resulted hair treatments were used, excellent use feelings were obtained in conditioning properties such as smoothness in rinse, excellent finger passing when wet and settlement of hair when dry.

**Table 3**

| Hair treatment | | | | | |
|---|---|---|---|---|---|
| Component | Example | | | | |
| | 6 | 7 | 8 | 9 | 10 |
| (component A) | | | | | |
| 2-guanidinoethylmyristylamid e phosphate | 2.0 | | | 3.0 | |
| 6-guanidinohexyldodecaneamid e lactate | | | 2.0 | | |
| 4-guanidinobutyldodecaneamid e acetate | | 2.0 | | | 5.0 |
| (component B) | | | | | |
| Nα-behenoylarginine | 0.1 | | | | 0.3 |
| Nα-decanoylarginine | | 1.0 | | 1.0 | |
| Nα-palmitoylarginine | | | 1.0 | | |
| (other components) | | | | | |
| Diglycerine | 2.0 | | 2.0 | | 2.0 |
| Alanine | | 2.0 | | 2.0 | |
| Isocetyl isostearate | 7.5 | 7.5 | 7.5 | 7.5 | 7.5 |
| Isostearyl alcohol | 10.0 | 10.0 | 10.0 | 10.0 | 10.0 |
| Polyoxyethylene tetraoleate sorbitol (EO40) | 0.5 | 0.5 | 0.5 | 0.5 | 0.5 |
| Polyoxyethylene hardened castor oil (EO40) | 1.0 | 1.0 | 1.0 | 1.0 | 1.0 |
| Polyoxyethylene hardened castor oil (EO60) | 1.0 | 1.0 | 1.0 | 1.0 | 1.0 |
| Trimethyl ammonium stearate chloride (50wt%, EtOH) | 3.0 | 3.0 | 3.0 | 3.0 | 3.0 |
| Butyl paravene | 0.1 | 0.1 | 0.1 | 0.1 | 0.1 |
| Purification water | Resid ue | Resid ue | Resid ue | resid ue | Resid ue |

### Examples 11 to 13

Self rinsing shampoos (2 in 1 shampoo) having compositions (wt% of net composition, total 100%) shown in the following Table 4 were prepared. When the resulted self rinsing shampoos were used, excellent use feelings were obtained in conditioning properties such as smoothness in rinse, excellent finger passing when wet and settlement of hair when dry.

**Table 4**

| Self rinsing shampoo(2 in 1 shampoo) | | | |
|---|---|---|---|
| Component | Example | | |
| | 11 | 12 | 13 |
| (component A) | | | |
| 4-guanidinobutylmyristylamide acetate | 1.0 | | |
| 4-guanidinobutylpalmitylamide sulfate | | | 1.0 |
| 3-guanidinopropyldodecaneamide laurate | | 1.0 | |
| (component B) | | | |
| Nα-cocoylarginine | 1.0 | | |
| Nα-lauroylarginine | | 1.0 | |
| Nα-myristoylarginine | | | 1.0 |
| (other components) | | | |
| Imidazolium betaine type amphoteric surfactant | 16.0 | 16.0 | 16.0 |
| coconut oil fatty acid diethanolamide | 4.0 | 4.0 | 4.0 |
| Coconut oil fatty acid acyl-L-arginineethyl DL-pyrrolidone carboxylate | 1.0 | 1.0 | 1.0 |
| stearyl trimethyl ammonium chloride | 2.0 | 2.0 | 2.0 |
| N-lauroyl-N-methyl-β-alanine sodium | 1.0 | 1.0 | 1.0 |
| POE alkylpolyamine | 1.0 | 1.0 | 1.0 |
| Aromatic | Suitable amount | Suitable amount | Suitable amount |
| Coloring matter | Suitable amount | Suitable amount | Suitable amount |
| Sodium hydroxide | Suitable amount | Suitable amount | Suitable amount |
| Purification water | Residue | Residue | Residue |

### Examples 14 to 16

Hair dyes having compositions (wt% of net composition, total 100%) shown in the following Table 5 were prepared. When the resulted hair dyes were used, excellent use feelings were obtained in conditioning properties such as smoothness in rinse, excellent finger passing when wet and settlement of hair when dry.

**Table 5**

| Hair dye | | | |
|---|---|---|---|
| Component | Example | | |
| | 14 | 15 | 16 |
| (component A) | | | |
| 6-guanidinohexylpalmitylamide citrate | 0.5 | | 0.6 |
| 4-guanidinobutylmyristylamide malate | | 0.7 | |
| (component B) | | | |
| Nα-palmitoylarginine | | 1.0 | 0.5 |
| Nα-stearoylarginine | 1.01 | | 0.5 |
| (other components) | | | |
| glycine betaine | 1.0 | 1.0 | 1.0 |
| p-phenylenediamine 3 | 3.0 | 3.0 | 3.0 |
| Resorcin | 0.2 | 0.2 | 0.2 |
| oleic acid | 20.0 | 20.0 | 20.0 |
| POE (10) oleyl alcohol ether | 13.0 | 13.0 | 13.0 |
| Isopropyl alcohol | 10.0 | 10.0 | 10.0 |
| Ammonia water (28%) | 10.0 | 10.0 | 10.0 |
| Antioxidant | Suitable amount | Suitable amount | Suitable amount |
| Chelating agent | Suitable amount | Suitable amount | Suitable amount |
| Purification water | Residue | Residue | Residue |

### Example 17

Hair treatment having compositions (wt% of net composition, total100%) shown in the following table6 were prepared. When the resulted hair rinse were used, excellent use feeling were obtained in conditioning properties such as smooth in rinse, excellent finger passing, when wet and settlement of hair when dry.

**Table 6**

| Hair rinse | |
|---|---|
| Component | Example 17 |
| (component A) | |
| 4-guanidinobutyldodecaneamide acetate 1 | |
| (component B) | |
| Nα-lauroylarginine | 0.05 |
| (other component) | |
| 4-aminobutyldodecaneamide acetate | 0.01 |
| 4-ureidobutyldodecaneamide | 0.01 |
| Nα,Ng-dilauroylagmatine | 0.02 |
| Silicone oil | 3 |
| Liquid paraffin | 1 |
| Cetyl alcohol | 1.5 |
| Stearyl alcohol | 1 |
| Glycerin | 0.7 |
| Perfume, color, perservative | suitable amount |
| Purification water | Residue |

The hair cosmetic of the present invention can improve overall conditioning properties such as smoothness in rinsing, excellent finger passing when wet, and settlement of hair when dry, by compounding one or more components selected from amide group-containing guanidine derivatives and salts thereof and one or more components selected from Nα-acylarginines and salts thereof, in combination. The hair cosmetic of the present invention having such a feature is extremely useful in shampoo, rinse, hair restoration agent, treatment, hair conditioner, tick, hair liquid, set lotion, permanent wave liquid, hair cream, hair lotion, hair mousse, permanent liquid, hair dye, hair color, hair manicure, and the like.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

## Claims

1. A hair cosmetic comprising, at least, one or more components A selected from amide group-containing guanidine derivatives of the following general formula (I), 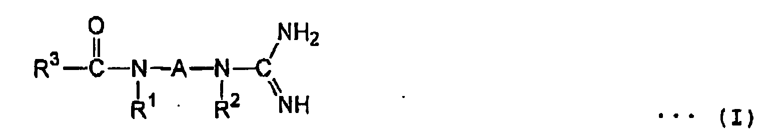 wherein R¹ and R² represent a hydrogen atom, or a linear or branched alkyl or alkenyl group having 1 to 4 carbon atoms, and may be the same or different, R³ represents a linear or branched alkyl or alkenyl group having 1 to 22 carbon atoms, and A represents a linear or branched alkylene or alkenylene group having 1 to 10 carbon atoms,
and salts thereof, and one or more components B selected from Nα-acylarginines of the following general formula (II), 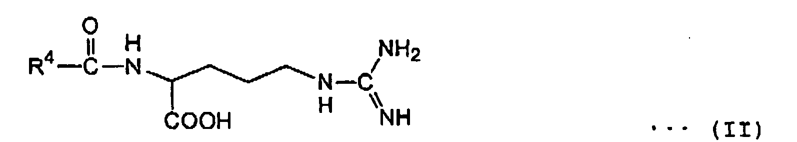 wherein R⁴ represents a linear or branched alkyl or alkenyl group having 1 to 22 carbon atoms,
and salts thereof.

## Patentansprüche

1. Haarkosmetikum, umfassend zumindest:
eine oder mehrere Komponenten A, ausgewählt aus den eine Amidgruppe enthaltenden Guanidinderivaten der nachstehenden Formel (I): 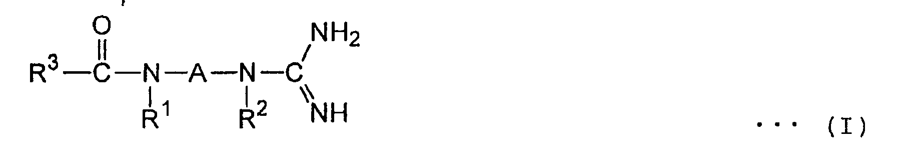 worin R¹ und R² für ein Wasserstoffatom oder eine unverzweigte oder verzweigte Alkyl- oder Alkenylgruppe mit 1 bis 4 Kohlenstoffatomen stehen und gleich oder unterschiedlich sein können, R³ für eine unverzweigte oder verzweigte Alkyl- oder Alkenylgruppe mit 1 bis 22 Kohlenstoffatomen steht und A für eine unverzweigte oder verzweigte Alkylen- oder Alkenylengruppe mit 1 bis 10 Kohlenstoffatomen steht,
und Salzen davon,
und eine oder mehrere Komponenten B, ausgewählt aus Nα-Acylargininen der nachstehenden Formel (II): 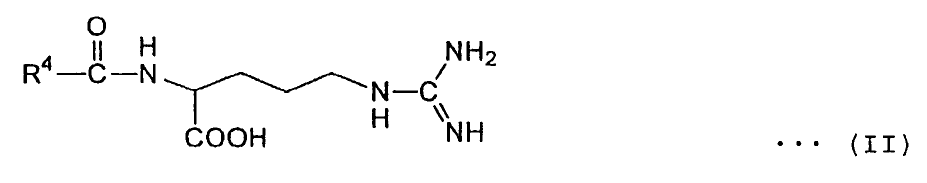
worin R⁴ für eine unverzweigte oder verzweigte Alkyl- oder Alkenylgruppe mit 1 bis 22 Kohlenstoffatomen steht,
und Salzen davon.

## Revendications

1. Produit capillaire cosmétique contenant, au moins, un ou plusieurs composants A sélectionnés parmi des dérivés de guanidine contenant un groupe amide de la formule générale suivante (I), 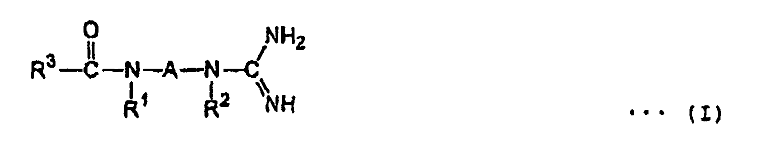 où R¹ et R² représentent un atome d'hydrogène, ou un groupe alkyle ou alkényle linéaire ou ramifié ayant 1 à 4 atomes de carbone, et peuvent être les mêmes ou différents, R³ représente un groupe alkyle ou alkényle linéaire ou ramifié ayant 1 à 22 atomes de carbone et A représente un groupe alkylène ou alkénylène linéaire ou ramifié ayant 1 à 10 atomes de carbone, et des sels de ceux-ci, et un ou plusieurs composants B sélectionnés parmi des Nα-acylarginine de la formule générale suivante (II), 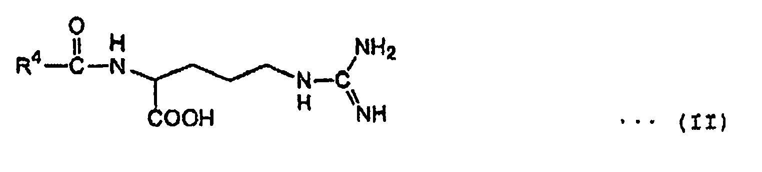 où R⁴ représente un groupe alkyle ou alkényle linéaire ou ramifié ayant 1 à 22 atomes de carbone, et ses sels.
